# EUROPEAN PATENT APPLICATION

(11) **EP 1 228 766 A1**
(43) Date of publication of application: **07.08.2002**
(21) Application number: 01102236.5
(22) Date of filing: 31.01.2001
(51) Int. Cl.: A61K 38/17, A61K 38/45, A61K 39/395, G01N 33/68

(54) **PYK2 phosphorylation by HER3 induces tumor invasion**

(71) Applicant: Max-Planck-Gesellschaft zur Förderung der Wissenschaften e.V., 80539 München (DE)
(72) Inventor: The designation of the inventor has not yet been filed
(74) Representative: Weiss, Wolfgang, Dipl.-Chem. Dr.

(57) **Abstract**

The present invention relates to the use of a HER2 protein or a nucleic acid coding therefor as a target for the modulation of the mitogen-activated protein (MAP) kinase pathway. Further, the use of a PYK2 protein and a nucleic acid coding therefor as a target for the modulation of the MAP kinase pathway is described. By inhibiting HER3 kinase activity, the phosphorylation of PYK2 and thus the stimulation of the MAP kinase pathway is inhibited. The present invention is preferably suitable for applications, particularly diagnostic or medical applications, wherein an inhibition of the MAP kinase pathway is desired. Thus, the invention relates to novel methods for diagnosing, treating or preventing MAP kinase associated disorders such as tumors.

## Description

The present invention relates to the use of a HER2 protein or a nucleic acid coding therefor as a target for the modulation of the mitogen-activated protein (MAP) kinase pathway. Further, the use of a PYK2 protein and a nucleic acid coding therefor as a target for the modulation of the MAP kinase pathway is described. By inhibiting HER3 kinase activity, the phosphorylation of PYK2 and thus the stimulation of the MAP kinase pathway is inhibited. The present invention is preferably suitable for applications, particularly diagnostic or medical applications, wherein an inhibition of the MAP kinase pathway is desired. Thus, the invention relates to novel methods for diagnosing, treating or preventing MAP kinase associated disorders such as tumors.

Glioblastoma multiforme, the most malignant tumor in the primary central nervous system, arises from neoplastic transformation of glioblasts, type 1 and type 2 astrocytes. Developmentally, glioblasts migrate out of the subventricular zone of the brain into developing white matter, differentiating and proliferating en route (1, 2). This inherent ability of astrocytes to migrate represents a key feature of glioma malignancy, when transformed cells invade the surrounding tissue.

Among mitogens and survival factors that are involved in activation of astrocyte migration, e.g. TGF-α, TGF-β, b-FGF and EGF, neuregulins have a potent effect on proliferation and differentiation by activating the MAPK pathway through SHC and phosphatidylinositol-3-OH-kinase (PI₃-K) (3, 4). Four neuregulins, NRG1 to NRG4, comprise a family of structurally related glycoproteins that are produced by proteolytic processing of transmembrane precursors (5-11). The multitude of NRG1 isoforms, which include neu differentiation factor (NDF), neuronal acetylcholine receptor-inducing activity protein (ARIA), glial growth factor (GGF), sensorimotor-derived factor (SMDF) and the heregulins (HRGs) (12), reflects their multiple growth- and differentiation-regulating activities in a variety of different biological systems.

Expression of HRGs was detected in the central and peripheral nervous systems (13), where they exert biological activities at neuronal muscle and neuronal Schwann cell junctions, respectively. HRGs represent ligands for the receptor protein tyrosine kinase (RPTK) erbB-family members HER3 (erbB3) and HER4 (erbB4). The HER family also includes HER1 (EGFR) and HER2/neu. HER3 represents a RPTK whose kinase activity is presumably impaired due to two mutations in the kinase domain (14). Transmission of the mitogenic signal involves binding of HRG either to HER3 or to HER4, which in turn heterodimerize with HER2 and become transphosphorylated at their C-terminus by activated HER2 (4, 15). Signalling molecules PI₃-K, SHC and GRB7 bind to the phosphorylated C-terminus of HER3 and mediate the mitogenic signal to the Ras/Raf pathway (16). The HER2/HER3 complex possesses the highest mitogenicity among HER heterodimers, presumably due to its redirection to the recycling pathway after ligang binding, instead of being degraded like HER1 (17).

A recently identified member of the focal adhesion kinase family PYK2, also designated as FAK2, CAK-β, RAFTK or CADTK, was shown to be a link in MAPK activation induced by G protein-coupled receptors (18, 19, 20). Phosphorylation of PYK2 leads to recruitment of Src-family kinases and to activation of extracellular signal-regulated kinases (ERKs). PYK2 is predominantly expressed in the central nervous system and in cells and tissues derived from hematopoietic lineage, where it is mainly diffused throughout the cytoplasm and concentrated in the perinuclear region (21). PYK2 can be activated by a variety of stimuli that increase intracellular calcium levels (22), and also by stress factors (e.g hyperosmotic shock, UV, tumor necrosis factor α), thereby inducing Jun N-terminal kinase (23, 24). However, the molecular details of the PYK2 activation mechanism are unknown.

In this study we examined the role of PYK2 tyrosine phosphorylation in human glioma cell lines upon HRG stimulation. We investigated the mechanism by which PYK2 becomes phosphorylated, its role in the MAPK pathway and subsequent effects on tumor invasion. We show that a kinase activity of HER3 directly phosphorylates PYK2, which in turn amplifies mitogenic signals mediated by the MAPK pathway. Our data suggest a pivotal role for PYK2 as a regulator of the invasive capacity of glioma cells.

Thus, a first aspect of the present invention relates to the use of a HER3 protein as a target for the modulation of the MAP kinase pathway.

A further aspect of the present invention relates to the use of a nucleic acid encoding a HER3 protein or a nucleic acid complementary thereto as a target for the modulation of the MAP kinase pathway.

A third aspect of the present invention relates to the use of a PYK2 protein as a target for the modulation of the MAP kinase pathway.

A fourth aspect of the present invention relates to the use of a nucleic acid encoding a PYK2 protein or a nucleic acid complementary thereto as a target for the modulation of MAP kinase activity.

A fifth aspect of the present invention relates to a method for identifying novel modulators of MAP kinase pathway activity by screening for substances capable of inhibiting HER3 phosphorylation and/or HER3 kinase activity.

A sixth aspect of the present invention relates to a method for identifying novel modulators of MAP kinase pathway activity by screening for substances capable of inhibiting PYK2 phosphorylation and/or PYK2 kinase activity.

The terms "HER3" or "PYK2" proteins as used in the present application particularly encompass mammalian proteins such as proteins from man, mouse, rat, hamster, monkey, pig, etc. Especially preferred is a HER3 protein comprising:
a) the amino acid sequence as shown in Genbank Accession No. M34309 and published in (51) or
b) an amino acid sequence having an identity of at least 80%, particularly of at least 90% and more particularly of at least 95% thereto, wherein the amino acid sequence identity may be determined by a suitable computer program such as GCG or BLAST.

Further especially preferred is a PKY2 protein comprising:
a) the amino acid sequence as shown in Genbank Accession No. U33284 and published in (18) or
b) an amino acid sequence having an identity of at least 80%, particularly of at least 90% and more particularly of at least 95% thereto, wherein the amino acid sequence identity may be determined by a suitable computer program such as GCG.

Furthermore, the terms "HER3" and "PYK2" protein encompass recombinant derivatives or variants thereof as well as fragments thereof having biological activity. These derivatives, variants and fragments may be obtained as expression products from allelic variant genes or from recombinantly altered, e.g. modified or truncated genes and/or as products of proteolytic cleavage. The term "biological activity" in context with HER3 preferably comprises a kinase activity, e.g. a direct kinase activity for PYK2, or the capability of acting as an inhibitor, e.g. a competitive inhibitor of native HER3 having reduced or abolished kinase activity. Particularly important residues for HER3 kinase activity are tyrosine residues Y1257, Y1270 and/or Y1288. Thus, HER3 analogs wherein these residues have been deleted or replaced by other amino acid residues may be used as inhibitors of native HER3. In context with PYK2 the term "biological activity" preferably comprises the capability of being phosphorylated by HER3 and acting as a stimulator of MAP kinase pathway or the capability of acting as an inhibitor, e.g. as a competitive inhibitor for the MAP kinase stimulation having reduced or abolished kinase activity. A particularly important residue for PYK2 kinase activity is lysine (K) at position 457 (ATP-binding site). Such derivatives, variants and fragments are obtainable by recombinant expression of corresponding nucleic acids in a suitable host cell and obtaining the resulting expression products by known methods. The activity of the resulting expression products may be determined according to the methods described in the present application, particularly in the examples section.

The HER3 protein is encoded by a nucleic acid, which may be a DNA or an RNA. Preferably, the nucleic acid comprises:
a) the nucleic acid sequence as shown in Genbank Accession No. M34309 or complementary thereto,
b) a nucleic acid sequence corresponding to the sequence of (a) within the scope of degeneracy of the genetic code or
c) a nucleic acid sequence hybridizing under stringent conditions with the sequence of a) and/or b).

The PYK2 protein is encoded by a nucleic acid, which may be a DNA or an RNA. Preferably, the nucleic acid comprises:
a) the nucleic acid sequence as shown in Genbank Accession No. U33284 or complementary thereto,
b) a nucleic acid sequence corresponding to the sequence of (a) within the scope of degeneracy of the genetic code or
c) a nucleic acid sequence hybridizing under stringent conditions with the sequence of a) and/or b).

The term "hybridization under stringent conditions" according to the present application is used as described in Sambrook et al., Molecular Cloning, A Laboratory Manual, Cold Spring Harbor, Laboratory Press (1989), 1.101-1.104. Consequently, hybridization under stringent conditions occurs when a positive hybridization signal is still detected after washing for 1 h with 1 x SSC and 0.1% SDS at 55°C, preferably at 62°C and most preferably 68°C, in particular for 1 h in 0.2 x SSC and 0.1% SDS at 55°C, preferably at 62°C and most preferably at 68°C. A nucleotide sequence hybridizing under such washing conditions with a sequence as shown in the sequence listing or a complementary nucleotide sequence or a sequence within the scope of degeneracy of the genetic code is encompassed by the present invention.

The nucleic acid molecules of the invention may be recombinant nucleic acid molecules generated by recombinant methods, e.g. by known amplification procedures such as PCR. On the other hand, the nucleic acid molecules can also be chemically synthesized nucleic acids. Preferably, the nucleic acid molecules are present in a vector, which may be any prokaryotic or eukaryotic vector, on which the nucleic acid sequence is present preferably under control of a suitable expression signal, e.g. promoter, operator, enhancer etc. Examples for prokaryotic vectors are chromosomal vectors such as bacteriophages and extrachromosomal vectors such as plasmids, wherein circular plasmid vectors are preferred. Examples for eukaryotic vectors are yeast vectors or vectors suitable for higher cells, e.g. insect cells or mammalian cells, plasmids or viruses.

The native HER3 protein is capable of directly phosphorylating PYK2 and thereby stimulating the mitogenic activity mediated by the MAP kinase pathway. Thus, an inhibition of HER3 phosphorylation may lead to an inhibition of the MAP kinase pathway. Thus, a preferred embodiment of the present invention comprises reducing the amount and/or activity of a HER3 protein in a target cell or a target organism. This reduced amount and/or activity of HER3 may be accomplished by administering a HER3 inhibitor, particularly an inhibitor of the HER3 kinase activity. This inhibitor may be a low molecular substance or an anti HER3 antibody, e.g. a polyclonal antiserum, a monoclonal antibody, antibody fragments, recombinant antibodies etc. In a further preferred embodiment the invention comprises reducing the expression of HER3 in a target cell or a target organism. This reduction may be accomplished, e.g. by inhibiting transcription or translation of a native HER3 gene, e.g. by administering suitable antisense nucleic acid molecules.

Due to this biological activity, HER3 is a suitable target for the manufacture of agents for the diagnosis, prevention or treatment of a MAP kinase pathway associated disorder, particularly a MAP kinase pathway overactivity associated disorder. More preferably, HER3 is a target for the diagnosis, prevention or treatment of a PYK2 phosphorylation associated disorder. This disorder may be a hyperproliferative disease, which may be selected from inflammatory processes and tumors such as breast cancer, acute myeloid leukemia (AML) and particularly gliomas. Most preferably, the present invention comprises an inhibition of HER3 kinase activity in order to inhibit tumor invasion particularly in gliomas.

According to the present invention it was found that phosphorylation of the PYK2 protein turns on and amplifies mitogenicity mediated by the MAP kinase pathway. Thus, an inhibiton of PYK2 protein, particularly an inhibition of PYK2 phosphorylation may lead to an inhibition of the MAP kinase pathway. This inhibition may be accomplished by administering an inhibitor of PYK2, which may be a low molecular weight substance or an anti-PYK2 antibody as described above, or a HER3 analog capable of inhibiting the kinase activity of native HER3. Alternatively, the inhibition may be accomplished by administering a nucleic acid, e.g. an antisense nucleic acid. Thus, the amount and/or activity of PYK2 in a target cell or a target organism may be reduced and/or the expression of PYK2 in a target cell or in a target organism may be reduced. In an especially preferred embodiment a mutated PYK2 protein or nucleic acid coding therefor is administered, wherein said mutated PYK2 protein exhibits an at least partial loss of phosphorylation and/or kinase activity.

The administration of HER3 and/or PYK2 inhibitors is preferably in the form of a pharmaceutical composition which additionally comprises suitable pharmaceutically acceptable carriers or diluents. The composition may be an injectable solution, a suspension, a cream, an ointment, a tablet, etc. The composition is suitable for diagnostic or medical, e.g. preventive or therapeutic applications, particularly in the field of cancer. The dosage and mode of administration route depends on the type and severity of the disorder to be treated and may be determined readily by a skilled practician.

For example, the administration of antibodies may be carried out according to known protocols, e.g. as described in (52). The administration in form of nucleic acids may also be carried out in form of known protocols, such as described in (53).

The administration of HER3 and/or PYK2 inhibitors may be combined with the administration of other active agents, particularly anti-tumor agents, e.g. cytotoxic substances and MAP kinase inhibitors such as PD98059 and UO126.

Still a further embodiment of the present invention is a method of identifying novel modulators of MAP kinase pathway activity comprising screening for substances capable of inhibiting HER3 phosphorylation and/or HER3 kinase activity. HER3 inhibitors are preferably selected from anti-HER3 antibodies and low molecular weight compounds. Additionally, the present invention provides a method for identifying novel modulators of MAP kinase pathway activity comprising screening for substances capable of inhibiting PYK2 phosphorylation and/or PYK2 kinase activity. PYK2 inhibitors are preferably selected from low molecular weight substances.

The screening method may be a high-throughput screening assay, wherein a plurality of substances is tested in parallel. The screening assay may be a cellular assay or a molecular assay, wherein an interaction of a substance to be tested with HER3 and/or PYK2 phosphorylation or kinase activity is determined. The proteins may be provided in a cellular system, preferably a cellular system overexpressing HER3 and/or PYK2, HER3 and/or PYK2 containing cell fractions or substantially isolated and purified HER3 and/or PYK2 proteins or fragments thereof, wherein the proteins are capable of being phosphorylated and/or capable of kinase activity. Any active substance identified by this method, e.g. any substance which has inhibitory activity, may be used as a pharmaceutical agent or as a lead structure, which is further modified to improve pharmaceutical properties. It should be noted that any pharmaceutical use of a substance, which is identified by the method of the present invention, or any modified substance, which results from a lead structure identified by the method of the present invention, is encompassed by the subject matter of the claims.

The present invention is explained in more detail in the following figures and examples.

### Figure Legends

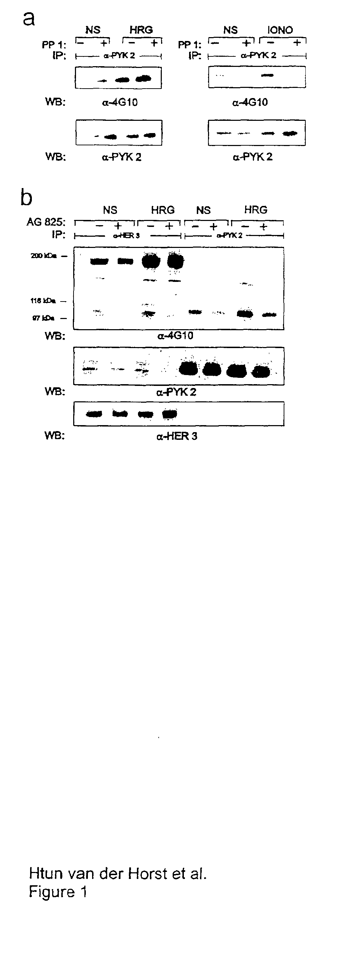

Figure 1 **Effects of a c-src inhibitor PP1 and a HER2 inhibitor AG825 on PYK2 tyrosine phosphorylation. a,** Tyrosine phosphorylation of PYK2 is independent of c-src upon HRG stimulation, in contrast to IONO stimulation. SF767 gliomas were pretreated with 5 µM PP1 for 30 minutes and stimulated either with 5 µg ml⁻¹ Heregulin (HRG, left panel) or 5 µM lonomycin (IONO, right panel) for 20 min and 5 min, respectively. b, PYK2 coprecipitation with HER3 depends on the HER2 kinase activity, and tyrosine phosphorylation of PYK2 is proportional to its binding to HER3. SF767 gliomas were pretreated with 10 µM AG825 for 1 hour and stimulated with 5 µg ml⁻¹ Heregulin for 20 min (HRG). Cell lysates were subjected to immunoprecipitation (IP) using polyclonal anti-PYK2 (α-PYK2) or monoclonal anti-HER3 (α-HER3) antibodies. Tyrosine phosphorylation level was analysed by western blotting (WB) with monoclonal anti-phosphotyrosine antibody (α-4G10) **(a,** upper panels, and **b,** upper panel). Equal loading of proteins was checked by reblotting with α-PYK2 and α-HER3 antibodies, respectively **(a,** lower panels, and **b,** middle and lower panels). PYK2 coprecipitating with HER3 was detected by probing the membrane with α-PYK2 antibody (**b**, middle panel, lanes 1-4). Unstimulated cells are indicated by NS.

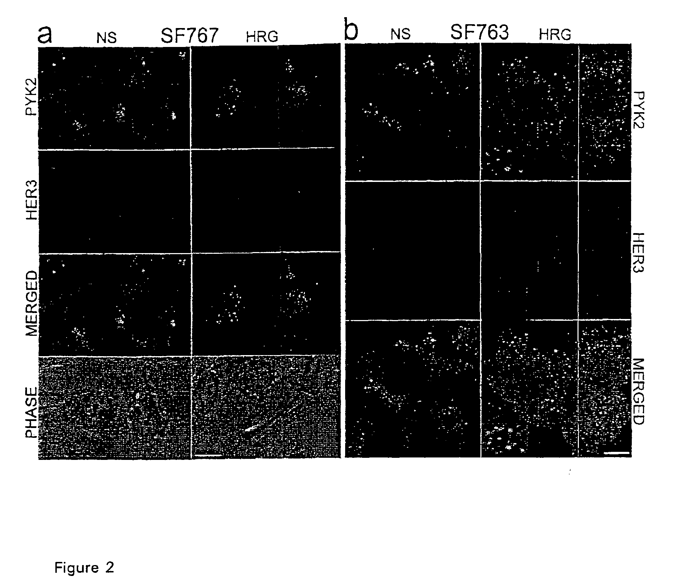

Figure 2 **Localization of PYK2 and HER3 in SF763 and SF767 glioma cell lines. a, b,** In SF767 **(a)** and in SF763 cells **(b),** PYK2 shows a punctated distribution throughout the cytoplasm, and is enriched in the perinuclear region and in some prominent cell protrusions (green). HER3 (red) is largely colocalized, as shown by overlapping distributions of the two stains in most puncta (**b**, insets) and in larger aggregates (yellow). Colocalization is independent of stimulation by HRG. Cells were fixed and immunostained against PYK2 (green) and HER3 (red), either unstimulated (NS) or following stimulation with 5 µg ml⁻¹ Heregulin for 20 min (HRG). Optical sections obtained by confocal laser scanning microscopy are shown. Scale bar represents 10 µm.

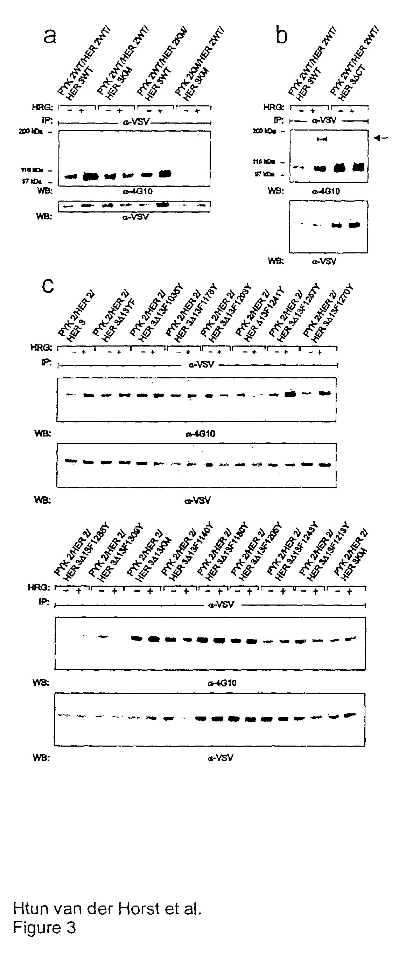

Figure. 3 **Association of PYK2 with the C-terminal domain of HER3. a, b, c,** HEK293 fibroblasts were either transfected with combinations of wild-type proteins (HER2, HER3, PYK2) and their dominant-negative variants (HER2-KM, HER3-KM, PYK2-KM) (**a**), with wild-type HER2 and PYK2 combined with wild-type HER3 or its truncated construct HER3ΔCT (**b**), or with wild-type HER2 and PYK2 and add-back mutants of HER3 (**c**), as indicated. Tyrosine phosphorylation of PYK2 is dependent on HER2 and HER3 kinase activity (**a**), and on binding to the C-terminal domain of HER3 (**b**). Coprecipitated HER3 is indicated by an arrow. PYK2 activation is dependent on Y1257, Y1270 and Y1288 in the C-terminal domain of HER3 (**c**). PYK2 was expressed tagged at its C-terminus with the vesicular somatitis virus glycoprotein (VSV). Cells were stimulated with 5 µg ml⁻¹ Heregulin for 20 min (HRG), lysed and subjected to immunoprecipitation with monoclonal anti-VSV antibody (α-VSV). Immunocomplexes were analysed by western blotting (WB) with a monoclonal anti-phosphotyrosine antibody (α-4G10, upper panels). Equal loading of proteins was determined by reblotting with α-VSV antibody (lower panels).

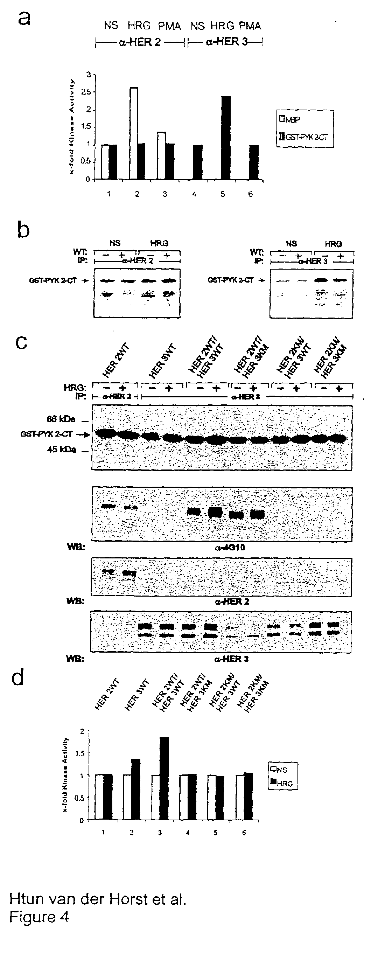

Figure 4 **Phosphorylation of GST-PYK2-CT by HER3 upon HRG stimulation. a, b,** SF767 gliomas were either stimulated with 5 µg ml⁻¹ Heregulin for 20 min (HRG) or with 1 µM Phorbol-12-myristate-13-acetate for 10 min (PMA) (**a**), or were pretreated with 100 nM Wortmannin for 30 min (WT) (**b**). PMA stimulation was used as a negative control. Note that kinase activity of HER3 is under 1 % of the corresponding HER2 activity when using MBP as a substrate, in contrast to GST-PYK2-CT (**a**). Upon HRG stimulation, phosphorylation of GST-PYK2-CT by HER3 is upregulated, in contrast to HER2 activity. Influence of WT is negligible, thus excluding involvement of PI₃-K in PYK2 phosphorylation (**b**). **c,** HEK293 fibroblasts were transfected with the combinations of wild-type proteins (HER2, HER3) and their dominant-negative variants (HER2-KM, HER3-KM) as indicated, and stimulated with 5 µg ml⁻¹ Heregulin for 20 min (HRG). Only homodimers of HER3 and heterodimers of HER3 with HER2 induced an increased GST-PYK2-CT phosphorylation (**c**, upper panel). Heterodimerization of HER3 with HER2 leads to a stronger phosphorylation of the substrate, indicating that HER2 is important for HER3 activation (**c** and **d**). Transphosphorylation of HER3 by HER2 was checked by probing the membrane with an anti-phosphotyrosine antibody α-4G10 (**c,** upper middle panel). Coprecipitation of HER2 with HER3 was excluded by probing the membrane with anti-HER2 antibody α-HER2 (**c**, lower middle panel). Equal loading of proteins was checked by probing with anti-HER3 antibody (α-HER3) (**c**, lower panel). Phosphorylated GST-PYK2-CT is indicated by an arrow. **d**, Quantification of the kinase activity shown in the upper panel of Fig. 4c.

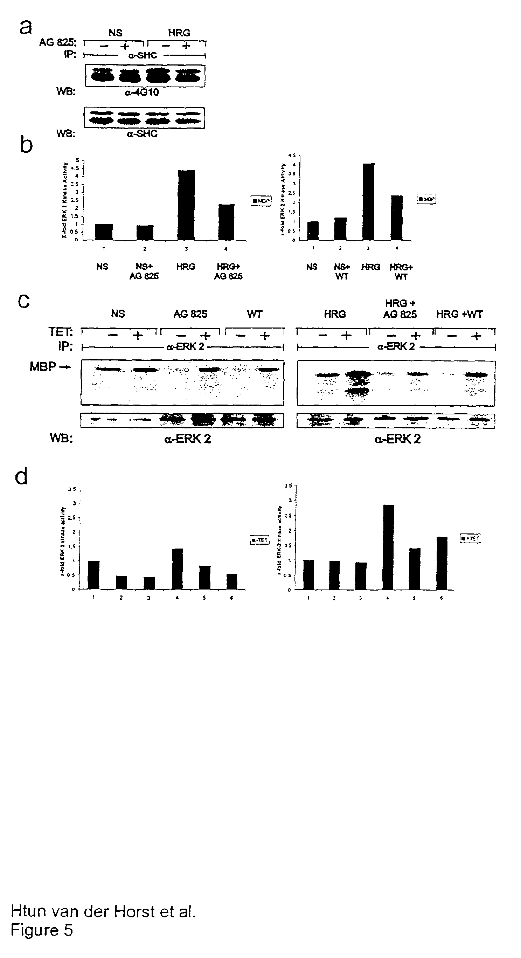

Figure 5 **PYK2 mediates mitogenicity upon HRG stimulation. a, b,** SF767 gliomas were pretreated either with 10 µM AG825 for 1 hour or with 100 nM Wortmannin for 30 min (WT), and then stimulated with 5 µg ml⁻¹ Heregulin for 20 min (HRG). Tyrosine phosphorylation of SHC was elevated by HRG and attenuated by pretreatment with AG825, but not fully abrogated (**a**). The same holds also for ERK-2 activity, when cells were pretreated either with AG825 or with WT (**b**). Cell lysates were used for immunoprecipitation with polyclonal anti-SHC (α-SHC) (**a**), or polyclonal anti-Erk-2 (α-ERK-2) antibodies (**b**). α-SHC-immunocomplexes were blotted with a monoclonal anti-phosphotyrosine antibody (α-4G10) (**a**), whereas α-ERK-2 immunocomplexes were subjected to MAP-kinase assays (**b**). Phosphorylated MBP is indicated by an arrow. **c**, Tetracyclin-inducible pheochromocytoma PC12 cells, either stably expressing PYK2-KM (Tet-), or only endogenous PYK2 (Tet +), were pretreated either with 100 nm Wortmannin for 30 min (WT), or 10 µM AG825 for 1 hour prior to stimulation with 5 µg ml⁻¹ Heregulin for 20 min (HRG). Basal ERK-2 activity is independent of HER2 and PI₃-K, whereas the HRG-stimulated ERK-2 activity is dependent on HER2,PI₃-K and PYK2. Overexpression of PYK2-KM leads to a general attenuation of ERK-2 activity (compare Tet- with Tet + bands). Equal loading of proteins was checked by probing with anti-ERK-2 antibody (α-ERK-2). Phosphorylated MBP is indicated by an arrow. **d**, Quantification of the ERK-2 kinase activity shown in Fig. 5c.

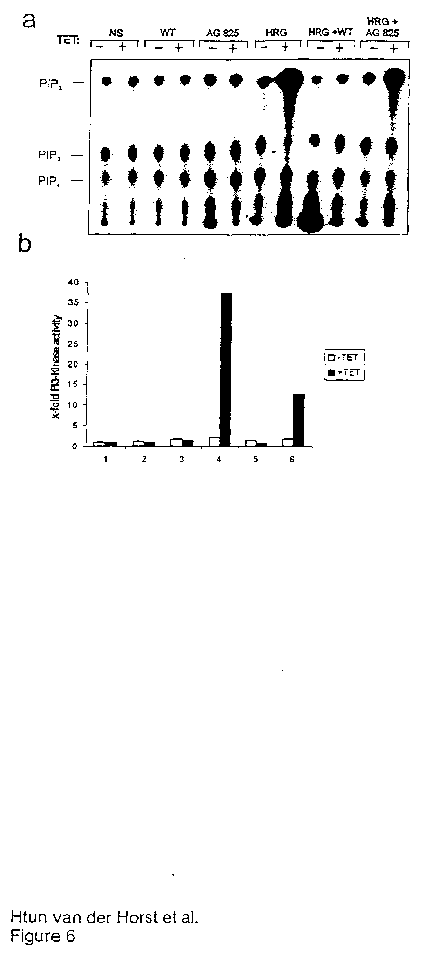

Figure 6 **PYK2 enhances PI**_{**3**}**-K activity upon HRG stimulation. a,** Tetracyclin-inducible pheochromocytoma PC12 cells, either stably expressing PYK2-KM (Tet-) or only endogenous PYK2 (Tet +), were pretreated either with 100 nm Wortmannin for 30 min (WT), or 10 µM AG825 for 1 hour prior to stimulation with 5 µg ml⁻¹ Heregulin for 20 min (HRG). Lysates were subjected to α-4G10 immunoprecipitation and PI₃-K assays were performed (see Methods section). PI₃-K activity is strongly dependent on PYK2 upon HRG stimulation, and is diminished by AG825. Phosphorylated Phosphatidylinositol is indicated. **b**, Quantification of the PI₃-K kinase activity shown in Fig. 6a.

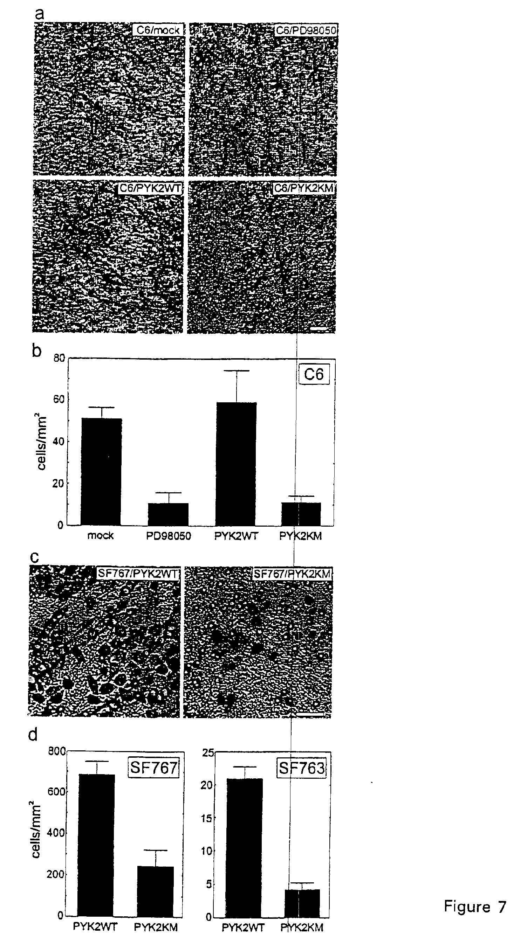

Figure 7 **PYK2-KM inhibits tumor invasion upon HRG stimulation. a,** C6 gliomas were retrovirally infected with either a control vector pLXSN (mock), PYK2, dominant negative PYK2 mutant PYK2-KM, or pretreated with a MEK1 inhibitor PD98059 (25 µM) for 30 min, and tumor invasion assays were performed (see Methods section). **b**, Invasion is supressed to the same extent by PD98059 and by overexpression of PYK2-KM (p>0.95). **c**, SF767 gliomas were retrovirally infected with pLXSN or PYK2-KM. **d**, Tumor invasion is supressed by overexpresssion of PYK2-KM in SF767 (p<0.008), and also in SF763 cell line (p<0.005), as shown by using the same assay. Representative bright-field micrographs of cells that migrated through the 8 µm filters in 16 h are shown. Scale bars represent 100 µm (**a**) and 50 µm (**c**).

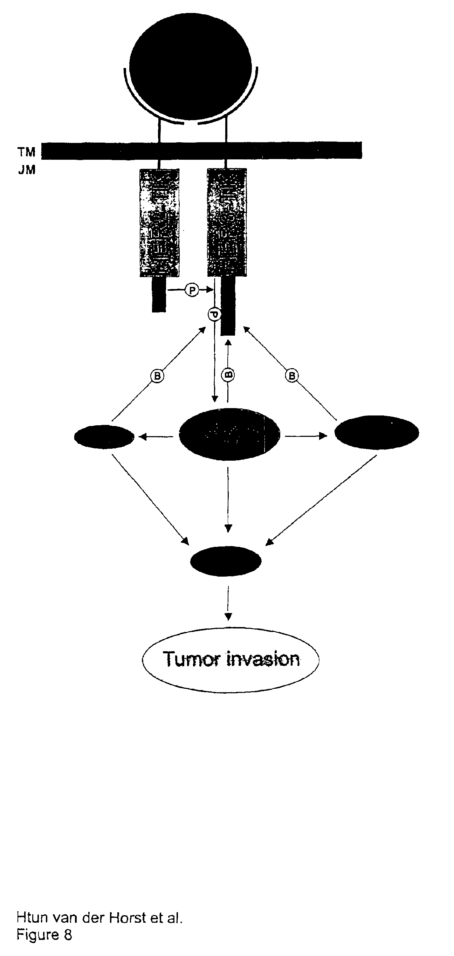

Figure 8 **Role of PYK2 in HER2/HER3 signalling.** Model indicates a novel signal transduction pathway, which leads from HRG stimulation to MAPK activation and induces tumor invasion. For details, see discussion. TM indicates the transmembrane domain, JM the juxtamembrane region. Arrows with an encircled B or P indicate binding and phosphorylation, respectively.

### Examples

### 1. Methods

### 1.1 Materials and general methods

Media were purchased from Gibco, fetal bovine serum (FBS) and horse serum from Sigma. Hybond ECL membranes and γ-³²P-ATP were purchased from Amersham, PP1, AG825 (ref. 45), Wortmannin (WT), PD98059 and lonomycin (IONO) from Calbiochem. Antibodies raised against following proteins were used: PYK2 (polyclonal goat antibody N19, Santa Cruz, and polyclonal rabbit antibody (pAb) Upstate Biotechnology, Inc. (UBI)), ERK2 (pAb C14, K23, Signal Transduction), SHC (pAb (ref. 46), mAb, Affiniti), HER3 (monoclonal mouse antibody (mAb) 2F12, UBI), p85 (mAb UB93-3, UBI), VSV (mAb P5D4, Roche Diagnostics), and phosphotyrosine (mAb 4G10, UBI). HRP-coupled secondary antibodies were purchased from Biorad, flourochrome-coupled secondary antibodies from Molecular Probes. Transwell chambers (0.3 cm², 8 µm) were purchased from Costar. Growth Factor Reduced Matrigel (GFRM) was purchased from Collaborative Biomedical Products. Thin-layer Chromatography plates (Silica Gel 60) precoated with oxalate were from Merck. Recombinant human GST-HRG fusion protein (HRG) and GST-PYK2-CT were produced in E. coli and purified as described (4) or using standard methods. Cell lines HEK293 (ATCC CRL-1573), rat C6 (ATCC CCL-107) and PC12 (ATCC CRL-1721) and human SF763 (Sugen Inc.), SF767 (Sugen Inc.) and PhoenixA (ATCC SD-3443) were cultured according to the supplier's protocol. Tetracyclin-inducible PC12 system stably expressing PYK2-KM (Tet-off) was described previously (25).

### 1.2 Immunofluorescence studies and confocal microscopy

Briefly, SF763 and SF767 (3x10⁵ cells) were grown on coverslips and starved for 24 h. After stimulation with 5 µg/ml HRG for 20 min, cells were fixed with 3.7% formaldehyd and permeabilized with 0.2% saponin (Sigma) in 3% BSA (Sigma). Blocking was performed with 3% BSA for 1 h.

PYK2 and HER3 proteins were labeled with the indicated primary antibodies and stained using a fluorochrome-coupled donkey anti-goat α-488 secondary antibody for PYK2, and TRITC-coupled rabbit anti-mouse secondary antibody for HER3 (Molecular Probes). Confocal microscopy was performed using an LSM 410 microscope (Zeiss) as described (47).

### 1.3 Plasmid constructs and site-directed mutagenesis

pcDNA3.1-PYK2-VSV and pcDNA3.1-PYK2-KM-VSV constructs were generated using the pRK5 constructs and standard methods. PYK2-KM was generated as described (25). GST-PYK2-CT was generated by using the pRK5 construct and amplifying the C-terminus of PYK2 by PCR (positions 716-1009). The fragment was subcloned into the procaryotic expression vector pGEX-5X1 (Pharmacia). Tyrosine to phenylalanine mutations in HER3 were performed using the pcDNA3.1-HER3 construct and the QuickChange site-directed mutagenesis kit (Stratagene) according to the manufacturers protocol. Correct incorporation of the mutations was verified by DNA sequencing.

### 1.4 Transient overexpression of PYK2, PYK2-KM, HER2, HER2-KM, HER3, and HER3-KM proteins in eukaryotic cells

The HEK293 cell system was used for transient protein expression. HEK293 cells were maintained in DMEM supplemented with 10% FCS, penicillin and streptomycin (100 IU/ml) at 7.5% CO₂ and 37°C. Transfections were carried out using a modified calcium phosphate method (48). Briefly, 2.5x10⁵ cells were incubated overnight in 3 ml of growth medium. 1 µg of supercoiled DNA was mixed with 0.25 M CaCl₂ solution in a final volume of 400 µl. The mixture was added to the same volume of 2x transfection buffer (50 mM BES, pH 6.95, 280 mM NaCI, 1.5 mM Na₂HPO₄) and incubated for 15 min at room temperature before it was added dropwise to the cells. After incubation for 12 h at 37°C under 3% CO₂, the medium was removed, cells were washed twice with PBS and were then starved for 24 h in DMEM supplemented with 0.1 % FCS.

### 1.5 Western Immunoblotting

SF763, SF767 or transfected HEK293 cells were either left untreated or were pretreated with PP1 (10 µM), AG825 (10 µM), Wortmannin (WT) (100 nM) and PD98059 (25 µM) for 30-60 min following stimulation with 5 µg/ ml recombinant human HRG for 20 min or with 5 µM IONO for 5 min at 37°C. Upon HRG or IONO stimulation, the cells were lysed on ice in a lysis buffer (50 mM HEPES pH 7.5, containing 150 mM NaCI, 1 mM EDTA, 10% (v/v) glycerol, 1% (v/v) Triton X-100, 1 mM sodium fluoride, 1 mM phenylmethylsulfonyl fluoride, 1 mM sodium orthovanadate, 1 mM β-glycerolphosphate, 10 mg/ml aprotinin). Crude lysates were centrifuged at 12500 g for 20 min at 4°C. For immunoprecipitations, the appropriate antiserum and 30 µl of protein A-Sepharose (Pharmacia) was added to the cleared lysate and incubated for 3 h at 4°C. Immunoprecipitates were washed with a washing buffer (20 mM HEPES pH 7.5, containing 150 mM NaCI, 1 mM EDTA, 1 mM Sodiumflouride 10% (v/v) glycerol, 1% (v/v) Triton X-100). Sample buffer containing SDS and 2-mercaptoethanol was added and the samples were denaturated by heating at 95°C for 4 min.

Proteins were fractionated by SDS-PAGE and electrophoretically transferred to nitrocellulose filters. For immunoblot analysis, nitrocellulose filters were first incubated with mouse monoclonal or rabbit polyclonal primary antibodies for 3 h at 4°C. Next, a HRP-coupled goat anti-mouse or goat anti-rabbit secondary antibody was added (Biorad), followed by an enhanced chemoluminescence (ECL) substrate reaction (Amersham). The substrate reaction was detected on Kodak X-Omat film. Filters that were used more than once with different antibodies were stripped according to the manufacturer's protocol, blocked and reprobed.

### 1.6 Generation of recombinant retroviruses and retrovirus-mediated gene transfer

Briefly, pLXSN-PYK2 and pLXSN-PYK2-KM were generated by cloning an EcoRI-XhoI fragment from pRK5 carrying the cDNAs of WT PYK2 and kinase-inactive PYK2, K457M (PYK2-KM), respectively, into pLXSN. Amphotrophic virus titer, which was generated by transient transfection of retrovirus expression plasmids into the virus producer cell line PhoenixA (ATCC), was determined by infecting NIH-3T3 cells with serial dilutions of retrovirus-containing, cell-free PhoenixA supernatants and counting the number of G418-resistant colonies. The titers were approximately 1x10⁶ cfu/ml both for PYK2 and PYK2-KM virus supernatants. Subconfluent C6, SF763 and SF767 cells (9x10⁵ cells) were incubated with supernatants of cells releasing high titers of pLXSN-PYK2 or pLXSN-PYK2-KM viruses (1x10⁶ G418 cfu/ml) for 24 h in the presence of Polybrene (4 mg/ml, Aldrich).

### 1.7 In-vitro-kinase assay

MAP-kinase and PI3-kinase assays were performed as described previously (49, 50).

### 1.8 Tumor invasion assay

Tumor invasion assay was performed as described previously (31). Briefly, 3x10⁵ cells were plated on transwell chambers precoated with 100 µg GFRM. Conditioned NIH-3T3 medium was used as a chemoattractant. Cells were stimulated with 5 µg/ml HRG during the experiment. Following 16 h of incubation, non-invading cells were removed with cotton swabs, whereas invading cells were fixed, stained with Crystal violet and counted under bright-field illumination using an Axiovert135 inverted microscope (Zeiss). Counts from 4 filters for each strain were pooled and compared among different strains using the two-tailed t-test.

### 2. Results

### 2.1 Tyrosine-phosphorylation of PYK2 is dependent on HER2 and HER3

PYK2 gets tyrosine-phosphorylated in human glioma cell line SF767 upon stimulation by HRG (Fig. 1a). In order to evaluate the mechanism of HRG-induced PYK2 tyrosine-phosphorylation, we inhibited two candidate protein tyrosine kinases, c-src and HER2. It has previously been reported that c-src kinase associates with HER2 after HRG-stimulation and phosphorylates PYK2 upon GPCR stimulation (20). C-src-inhibition with PP1 prior to HRG stimulation indicates that c-src does not mediate PYK2 tyrosine-phosphorylation after HRG treatment. In contrast, stimulation by lonomycin, which leads to an influx of Ca²⁺-ions analogously to a GPCR stimulation (25), induces a tyrosine-phosphorylation of PYK2 that is dependent on c-src activity (Fig. 1a, left vs. right panel, lanes 3 and 4).

In the breast carcinoma cell line MDA-MB-435 it has been shown that HRG-induced activation of HER2, which is mediated by heterodimerization between HER2 and HER3, leads to tyrosine-phosphorylation of PYK2 (26). A tyrosine phosphorylated protein of Mᵣ = 113 kDa, which we identified as PYK2, coprecipitates with HER3 in SF767 cells prior to stimulation with HRG (Fig. 1b, upper and middle panels, lanes 1-4). In contrast, precipitation of HER2 reveales no association with PYK2. Upon HRG-stimulation tyrosine-phosphorylation of PYK2 increases (Fig. 1b, upper panel, lanes 5 and 7), but is attenuated in presence of HER2 inhibitor AG825 (Fig. 1b, upper panel, lanes 6 and 8), indicating that tyrosine-phosphorylation of PYK2 is dependent on the HER2 kinase activity. The amount of PYK2 that coprecipitates with HER3 is not elevated by HRG-stimulation (Fig. 1b, middle panel, lanes 1 and 3), but decreases after addition of AG825 (Fig. 1b, middle panel, lanes 2 and 4). The same results were also obtained in the glioma cell line SF763, and suggest a constitutive association of PYK2 with HER3, which is dependent on the HER2 kinase.

To further analyse a cellular colocalization of PYK2 and HER3, we performed immunoflourescence studies in SF763 and SF767 cell lines using a laser scanning confocal microscope (Fig. 2). In unstimulated cells PYK2 is mainly localized to the perinuclear cytoplasm in a punctuated pattern, and distribution of HER3 is largely coincident. Upon stimulation with HRG, the colocalization of PYK2 with HER3 remained unchanged. Thus, immunofluorescence studies confirmed a constitutive, HRG-independent association between PYK2 and HER3.

### 2.2 PYK2 associates with the intracellular region of HER3

We used an ectopic overexpression system to investigate in detail how tyrosine-phosphorylation of PYK2 depends on binding to HER3. HEK293 fibroblasts were used to express either wild-type HER2, HER3 and PYK2 or dominant-negative mutant constructs HER2-KM, HER3-KM and PYK2-KM, where the lysine critical for ATP-binding was exchanged to alanine, rendering the kinase inactive. Tyrosine-phosphorylation of PYK2 was elevated upon HRG-stimulation of cells expressing all the wild-type constructs (Fig. 3a, lanes 1 and 2). However, in cells expressing HER3-KM (Fig. 3a, lanes 3 and 4) or HER2-KM (Fig. 3a, lanes 5 and 6), HRG-stimulation failed to induce PYK2 tyrosine-phosphorylation. This observation is consistent with the data from glioma cell lines (Fig. 1b), where inhibition of HER2 abrogated PYK2 activation, but further implies that HRG-induced PYK2 activation is dependent on functional kinase activities of HER2 and HER3.

Next we used a mutant of HER3 with a C-terminal deletion (HER3ΔCT) to analyse the contribution of the C-terminal domain to HRG-induced PYK2 tyrosine-phosphorylation (Fig. 3b). We observed a coprecipitating protein of Mᵣ = 180 kDa in PYK2 immunocomplexes, which was phosphorylated and confirmed to be HER3 (Fig. 3b, lanes 1 and 2). The deletion mutant of HER3 abrogated the tyrosine-phosphorylation of PYK2 and also coprecipitation of HER3, indicating that PYK2 associates with the C-terminal region of HER3 (Fig. 3b, lanes 3 and 4). The intracellular domain of HER3 harbours 13 phosphorylation sites that are presumably transphosphorylated by HER2 after HRG-stimulation. The tyrosines Y1035, Y1178, Y1203, Y1241, Y1257 and Y1270 are potential docking sites for the src-homology 2 (SH2) domains of the regulatory domain p85 of PI₃-K (27), whereas Y1309 is a binding site for SHC (28). To identify the putative binding sites for PYK2 on the C-terminal domain of HER3, we used 13 add-back mutants, replacing all tyrosine residues to phenylalanines and exchanging each one back to a tyrosine. We performed overexpression experiments in HEK293 fibroblasts, using wildtype PYK2 and HER2, and single add-back mutants of HER3 (Fig. 3c). Using this approach, we identified three tyrosine residues Y1257, Y1270 and Y1288, which are critical for elevated PYK2 tyrosine-phosphorylation upon HRG-stimulation (Fig. 3c, lanes 13-18). These observations show that the HRG-induced stimulation of PYK2 tyrosine-phosphorylation depends on its binding to Y1257, Y1270 and Y1288 in the C-terminal domain of HER3.

### 2.3 Tyrosine-phosphorylation of PYK2 is dependent on HER3 kinase activity

It has been implied that, in contrast to HER2, kinase activity of HER3 is impaired (29), although HER3 can bind ATP and its analog TNP-ATP (30). To identify the kinase which is responsible for the PYK2 tyrosine-phosphorylation upon HRG-stimulation, we conducted *in vitro* kinase assays, precipitating either HER2 or HER3, using myelin basic protein (MBP) and a GST-fusion protein of the C-terminal region of PYK2 (GST-PYK2-CT) as substrates (Fig. 4a). Upon stimulation of SF767 cells either with HRG, or with Phorbol-12-myristate-13-acetate (PMA), MBP became phosphorylated by HER2, but not by HER3 (Fig. 4a, white bars). Surprisingly, however, GST-PYK2-CT became phosphorylated by HER3 in a HRG-stimulation-dependent way, but not by HER2 (Fig. 4a, black bars). As it has been shown that PI₃-K binds to the cytoplasmic tail of HER3, we investigated a potential role of PI₃-K in PYK2 phosphorylation by precipitating either HER2 or HER3 in the presence or absence of PI₃-K-inhibitor Wortmannin (WT) (Fig. 4b). The results indicate that PI₃-K is not responsible for the direct phosphorylation of GST-PYK2-CT. Consistent with this finding, precipitation of PYK2 under the same experimental conditions showed that its elevated tyrosine-phosphorylation upon HRG-stimulation is independent of PI₃-K. Taken together, these data suggest that HER3 is the kinase which phosphorylates the C-terminal region of PYK2.

To verify that HER3 directly phosphorylates PYK2, we overexpressed HER2 and HER3 either separately, or together in combinations of wild-type constructs and dominant-negative mutants in HEK293 cells (Fig. 4c). Receptor-immunocomplexes were subjected to *in vitro* kinase assay and revealed that, after HRG-stimulation, HER3 phosphorylates GST-PYK2-CT, whereas HER2 does not (Fig. 4c, upper panel, lanes 3, 4, 5 and 6 vs. lanes 1 and 2). HER3 homodimers also phosphorylated GST-PYK2-CT, but to a lesser extent compared to transactivated HER3 (Fig. 4c, upper panel, lanes 3 and 4). To show that HER3 is transphosphorylated by HER2 upon HRG-stimulation, we probed with monoclonal phosphotyrosine antibody α-4G10 (Fig. 4c, middle upper panel). We also show that there was no significant coprecipitation of HER2 in the HER3 immunocomplex under our assay conditions, confirming that HER2 is not the kinase which phosphorylates GST-PYK2-CT (Fig. 4c, middle lower panel). We conclude that HER3 directly phosphorylates PYK2 upon HRG-stimulation.

### 2.4 PYK2 amplifies mitogenicity of the HER2/HER3 signalling pathway

Upon stimulation of HER3 and HER2, PI₃-K and SHC bind to the C-terminus of HER3 and mediate mitogenicity through the Ras/Raf pathway (15). To test the influence of HER2 and PI₃-K on MAPK activation, we added their specific inhibitors AG825 and Wortmannin (WT), respectively, to SF767 cells prior to HRG-stimulation. Then we precipitated SHC or performed MAP-kinase assays. HRG-stimulated tyrosine-phosphorylation of SHC and ERK-2 activity were diminished, but not fully abrogated by inhibition of HER2 (Fig. 5a, 5b, left panel). The analogous experiment using WT for inhibition of PI₃-K revealed that ERK-2 activity was reduced by WT (Fig. 5b, right panel). These findings indicate that HRG-induced mitogenicity only partially depends on HER2 and PI₃-K.

To characterize in more detail the role of PYK2 in signalling downstream of HER2/HER3, we used a tetracyclin-inducible system (Tet-off) in pheochromocytoma cell line PC12. PC12 cells are rich in PYK2, so that in the presence of Tet endogenous PYK2 is predominantly expressed, whereas its removal leads to overexpression of dominant-negative PYK2, PYK2-KM. We inhibited either HER2 or PI₃-K with AG825 and WT, respectively, prior to stimulation with HRG, precipitated ERK-2 and subjected the immunocomplexes to MAP-kinase assays (Fig. 5c). Basal ERK-2 activity was not influenced by AG825 and WT, but was abrogated by PYK2-KM expression. HRG-stimulated ERK-2 activity, however, was attenuated by the two inhibitors, and also abrogated by PYK2-KM expression. These findings are consistent with the results obtained in SF767 (Fig. 5b). Taken together, these results indicate that the constitutive ERK-2 activity depends on PYK2, and is independent of HER2 and PI₃-K, whereas HRG-stimulated ERK-2 activity depends on HER2 and PI₃-K, and also on PYK2.

In addition to its role in cell proliferation and in prevention of apoptosis, an influence of PI₃-K on carcinoma invasion has previously been shown (31). We therefore investigated the potency of PYK2 and its dominant-negative mutant PYK2-KM to regulate PI₃-K activation upon HRG-stimulation. Using the Tet-off system in PC12 cells we subjected cell lysates to PI₃-K assays, where we observed a PYK2-dependent PI₃-K activation upon HRG-stimulation (Fig. 6, upper panel, lanes 1 vs. 2 with 7 and 8). Inhibition of HER2 kinase activity did not fully abrogate PI₃-K activity, indicating a HER2-independent mechanism of PI₃-K activation (Fig. 6, upper panel, lanes 8 and 12). These results imply an important role of PYK2 in mediating mitogenicity to the MAPK signalling pathway, and in PI₃-K activation upon HRG-stimulation.

### 2.5 PYK2-KM inhibits tumor invasion by blocking mitogenicity of the HER2/HER3 signalling pathway

Gliomas represent a highly malignant brain tumor phenotype with a poor prognosis (32). It has been shown that PI₃-K links α6β4-integrin signalling to invasive behaviour of breast tumor cells (31). Further, it has been reported that activation of MAPK through α6β4-integrin signalling is relevant to invasion, due to its importance in migration and its ability to phosphorylate myosin light chain kinase (33). Using C6 gliomas as a model system for tumor invasion (34), we tested whether the dominant-negative mutant of PYK2, PYK2-KM, can inhibit tumor invasion by blocking the MAPK pathway. We retrovirally infected the cells with PYK2-KM prior to stimulation with HRG, and also pretreated the cells with the MEK1 inhibitor PD98059 (Fig. 7a). MEK1- inhibition strongly attenuated invasiveness and a comparable abrogation of the invasive phenotype was observed upon infection of cells with PYK2-KM. The mitogenic signal of the HER2/HER3 dimer seems to be downregulated by PYK2-KM, however, overexpression of PYK2 in C6 cells did not alter their invasive phenotype (Fig. 7b). PYK2 expression in C6 cells is comparably weaker than in SF763 or SF767 cells, but this does not seem to interfere with their invasive potency. We also tested glioma cell lines SF763 and SF767 in the tumor invasion assay, after viral infection with the PYK2-KM construct. Again, a strong inhibition of the invasive behaviour of tumor cells by PYK2-KM was observed (Fig. 7d). These results demonstrate that PYK2 can mediate mitogenicity through the MAPK pathway, which plays an important role in the invasive behaviour of gliomas upon HRG-stimulation.

### 3. Discussion

Cytoplasmic protein tyrosine kinase PYK2 is at the convergence point of transduction pathways that transmit signals from stimulated integrins, G protein-coupled receptors and PTK receptors to downstream effectors. An important stimulus that activates PYK2 is HRG (25). Both PYK2 and HRG are predominantly expressed in the central nervous system, and the genes coding for the two proteins are localized in the close proximity to each other on the chromosome 8 (34). HRG is a promiscuous ligand for HER3 and HER4, members of the erbB family of RPTKs, and the erbB signalling module represents one of the most potent inducers of mitogenicity (35). Binding of HRG leads to formation of HER2/HER3 and HER2/HER4 heterodimers, thereby activating HER2 which transphosphorylates HER3 or HER4 (35). Signalling molecules SHC and PI₃-K are known to bind to the C-terminal region of HER3 and to promote mitogenicity (4, 15, 35). These pieces of information, obtained in several model systems, prompted us to explore an HRG-stimulated signalling pathway involving HER2/HER3 and PYK2 in glioblastoma cell lines which are devoid of HER4. Based on the presented data, we propose a model in which PYK2 is phosphorylated by HER3 upon HRG stimulation, and induces invasiveness through the MAPK pathway (Fig.8).

Immunoprecipitation assays indicate a constitutive association of PYK2 with HER3, which is promoted by HER2 activity (Fig.1). Immunofluorescence studies confirmed the constitutive assocation, showing that the two proteins co-localize in a punctuated pattern throughout the cytoplasm independent of HRG stimulation (Fig.2). It is known that HER3 is internalized through the clathrin-mediated endocytotic pathway (17). Similar punctuated distributions have recently been shown for several proteins associated with this pathway, e.g. mHip1r and EGFR (36, 37). Centripetal movement of the clathrin-coated vesicles towards the perincular region, which occurs on the time scale of several minutes, has been directly demonstrated by using a GFP-chlatrin fusion in Dictyostelium and COS-1 cells (38, 39). A prolonged activation state of HER3/PYK2 complexes within endosomes during recycling would enable recurrent association of other signalling molecules and thus serve to amplify the initiating signal. This prolonged accessibility of HER3/PYK2 complexes and their transport towards the site of MAPK activity could explain the exceptionally strong mitogenicity of HER2/HER3 heterodimers, compared to other members of the erbB family (35). Indeed, it has been shown that HER2/HER3 heterodimers are getting recycled, whereas HER1-containing dimers are degraded via ubiquitination pathway (17).

Although association of PYK2 with HER3 and its recycling appear to be HRG-independent, tyrosine phosphorylation of PYK2 is induced by HRG stimulation. Our evaluation of the mechanism by which PYK2 is activated upon HRG stimulation shows that intact kinase activity of HER3 is critical for PYK2 activation (Fig.3). Specifically, tyrosine residues Y1257, Y1270 and/or Y1288 in the C-terminal region of HER3 are shown to be important for PYK2 activation. Finally, in vitro kinase assays showed that HER3 directly phosphorylates GST-PYK2-CT (Fig.4). By inhibition experiments, we could exclude HER2, c-src and PI₃-K as proteins that directly phosphorylate PYK2. The HER3 kinase activity has possibly not been unraveled until now because previous studies used artificial substrates (28).

We show a negative effect of dominant negative PYK2, PYK2-KM, on MAPK activation, demonstrating that mitogenicity depends on PYK2 activity (Fig.5). It has been shown that cells overexpressing PYK2 exhibit elevated tyrosyl phosphorylated SHC and subsequent ERK-2 activity (40). We did not observe a direct interaction between PYK2 and SHC, but it has been proposed recently that SHC associates with PYK2 through GRB2 in platelets dependent on αIIbβ3 integrin, thus linking extracellular signal to the Ras/Raf pathway (41). It is possible that GRF2 binds to activated PYK2, leading to subsequent tyrosine phosphorylation of SHC, which contributes to increased mitogenicity. We also show that PYK2-KM attenuates PI₃-K activity (Fig.6). HER3 harbours six potential docking sites for the SH2 domain of the PI₃-K subunit p85, and the one proline-rich sequence that forms a consensus binding site for the SH3 domain of p85, all potentially contributing to an association of HER3 with p85 (26). Also, a constitutive association between PYK2 and p85 in platelets was reported (42), where one YXXM motif in PYK2 could serve for binding to the SH2 domain of p85. Indeed, immunoprecipitation of p85 revealed HRG-dependent association of tyrosyl phosphorylated proteins of Mᵣ = 113 kDa and 180 kDa. These proteins were identified as PYK2 and HER3, suggesting that HER3, PYK2 and PI₃-K are constituents of a multiprotein complex.

In our model we propose that PYK2 is a key element in transmitting HRG-induced mitogenicity. Part of the singalling from PYK2 to ERK-2 seems to be transmitted through PI₃-K and SHC, but there is also a more direct pathway (Fig.8). The PYK2-dependent ERK-2 activation also seems to be partly independent of HER2 (Fig.5). These findings suggest that PYK2 is involved in the control of multiple downstream effectors, which in turn all influence the MAPK pathway (Fig.8).

We show that the dominant PYK2 mutant PYK2-KM suppresses tumor invasiveness in three glioma cell lines (Fig.7). This result correlates with its influence on ERK-2 activity (Fig.6). Also, PYK2-KM abrogated invasiveness to the same extent as inhibition of MEK1. These results strongly indicate that PYK2 regulates invasiveness in gliomas through the MAPK pathway. It has been shown that ERK activity can regulate myosin phosphorylation, leading to actin-myosin association and cell contraction of the ECM (43), and that ERK can facilitate cell invasion and protect cells from apoptosis (44). Increased MAPK activity showed in our case an increased invasive behavior. Taken together, we show for the first time that PYK2 is a direct substrate of HER3, potentiates PI₃-K activity and enhances mitogenicity through ERK2 and in gliomas, leading to a strongly invasive phenotype.

### Literature

1. Altman, J. Proliferation and migration of undifferentiated precursor cells in the rat during postnatal gliogenesis. *Exp. Neurol.* **15,** 263-278 (1966).
2. Goldman, J.E. Regulation of oligodendrocyte differentiation. *Trends Neurosci.* **15,** 359-362 (1992).
3. Faber-Elman, A., Solomon, A., Abraham, J.A., Marikovsky, M. & Schwartz, M. Involvement of wound-associated factors in rat brain astrocyte migratory response to axonal injury. In vitro simulation. *J. Clin. Invest.* ***97,*** 162-171 (1996).
4. Wallasch, C. et al. Heregulin-dependent regulation of HER2/neu oncogenic signaling by heterodimerization with HER3. *EMBO J.* **14,** 4267-4275 (1995).
5. Busfield, S.J. et al. Characterization of a neuregulin-related gene, Don-1, that is highly expressed in restricted regions of the cerebellum and hippocampus. *Mol. Cell. Biol.* **17,** 4007-4014 (1997).
6. Carraway, K.L. III et al. Neuregulin-2, a new ligand of ErbB3/ErbB4-receptor tyrosine kinases. *Nature* **387,** 512-516 (1997).
7. Chang, H., Riese, D.J., II, Gilbert, W., Stern, D.F. & McMahan, U.J. Ligands for ErbB-family receptors encoded by a neuregulin-like gene. *Nature* **387,** 509-512. (1997).
8. Fischbach, G.D. & Rosen, K.M. ARIA: a neuromuscular junction neuregulin. *Annu. Rev. Neurosci.* **20,** 429-458 (1997).
9. Zhang, D. et al. Neuregulin-3 (NRG3): a novel neural tissue-enriched protein that binds and activates ErbB4. *Proc. Natl. Acad. Sci. USA* **94,** 9562-9567 (1997).
10. Ishiguro, H. et al. Structure and function of a novel ErbB ligand, NTAK. *Nihon Shinkei Seishin Yakarigaku Zasshi* **18,** 137-142 (1998).
11. Harari, D. et al. Neuregulin-4: a novel growth factor that acts through the ErbB-4 receptor tyrosine kinase. *Oncogene* **18,** 2681-2689 (1999).
12. Holmes, W.E. et al. Identification of heregulin, a specific activator of p185erbB2. *Science* **256,** 1205-1210 (1992).
13. Orr-Urteger, A. et al. Neural expression and chromosomal mapping of Neu dedifferentiation factor to 8p12-p21. *Proc. Natl. Acad. Sci. USA* **90,** 1867-1871 (1993).
14. Plowman, G.D. et al. Molecular cloning and expression of an additional epidermal growth factor receptor-related gene. *Proc. Natl. Acad. Sci. USA* **87,** 4905-4909 (1990).
15. Alroy, I. & Yarden Y. The ErbB signaling network in embryogenesis and oncogenesis: signal diversification through combinatorial ligand-receptor interactions. *FEBS* **410,** 83-86 (1997).
16. Fiddes, R.J. et al. Analysis of Grb7 recruitment by heregulin-activated erbB receptors reveals a novel target selectivity for erbB3. *J. Biol. Chem.* **273,** 7717-7724 (1998).
17. Ceresa, B.P. & Schmid, S.L. Regulation of signal transduction by endocytosis. *Curr. Opin. Cell. Biol.* **12,** 204-210 (2000).
18. Lev, S. et al. Protein tyrosine kinase PYK2 involved in Ca²⁺-induced regulation of ion channel and MAP kinase functions. *Nature* **376,** 737-745 (1995).
19. Girault, J.A., Costa, A., Derkinderen, P., Studler, J.M. & Toutant, M. FAK and PYK2/CAKbeta in the nervous system: a link between neuronal activity, plasticity and survival? *Trends Neurosci.* **22,** 257-263 (1999).
20. Dikic, I., Tokiwa, G., Lev, S., Courtneidge, S.A. & Schlessinger, J. A role for Pyk2 and Src in linking G-protein-coupled receptors with MAP kinase activation. *Nature* **383,** 547-50 (1996).
21. Andreev, J. et al. Identification of a new Pyk2 target protein with Arf-GAP activity. *Mol. Cell. Biol.* **19,** 2338-2350 (1999).
22. Avraham, H., Park, S.-Y., Schinkmann, K. & Avraham, S. RAFTK/PYK2-mediated cellular signalling. *Cell. Signal.* **12**, 123-133(2000).
23. Yu, H. et al. Activation of a Novel Calcium-dependent Protein-tyrosine Kinase. *J. Biol. Chem.* **271,** 29993-29998 (1996).
24. Tokiwa, G., Dikic, I., Lev, S. & Schlessinger, J. Activation of Pyk2 by stress signals and coupling with JNK signaling pathway. *Science* **273,** 792-794 (1996).
25. Zwick, E., Wallasch, C., Daub, H. & Ullrich, A. Distinct Calcium-dependent pathways of epidermal growth factor receptor transactivation and PYK2 tyrosine phosphorylation in PC12 cells. *J. Biol. Chem.* **274,** 20989-20996 (1999).
26. Zrihan-Licht, S. et al. RAFTK/Pyk2 tyrosine kinase mediates the association of p190 RhoGAP with RasGAP and is involved in breast cancer cell invasion. *Oncogene* **19,** 1318-1328 (2000).
27. Hellyer, N.J., Cheng, K. & Koland, J.G. ErbB3 (HER3) interaction with the p85 regulatory subunit of phosphoinositide 3-kinase. *Biochem J.* **333,** 757-763, (1998).
28. Prigent, S.A. & Gullick, W.J. Identification of c-erbB-3 binding sites for phosphatidylinositol 3'-kinase and SHC using an EGF receptor/c-erbB-3 chimera. *EMBO J.* **13,** 2381-2841 (1994).
29. Guy, P.M., Platko, J.V., Cantley, L.C., Cerione, R.A. & Carraway, K.L.III. Insect cell-expressed p180erbB3 possesses an impaired tyrosine kinase activity. *Proc. Natl. Sci. USA* **91,** 8132-8136 (1994).
30. Sierke, S.L., Cheng, K., Kim, H.-H. & Koland, J.G. Biochemical characterization of the protein kinase homology domain of the ErbB3 (HER3) receptor protein. *Biochem. J.* **322,** 757-763 (1997).
31. Shaw, L.M., Rabinovitz, I., Wang, H.H., Toker, A., & Mercurio, A.M. Activation of phosphoinositide 3-OH kinase by the alpha6beta4 integrin promotes carcinoma invasion. *Cell* **91,** 949-60 (1997).
32. Berens, M.L. & Giese, A. Biology and oncology of invasive glioma cells. *Neoplasia* **3,** 208-219 (1999).
33. Klemke, R.L. et al. Regulation of cell motility by mitogen-activated protein kinase. *J Cell. Biol.* **137,** 481-492 (1997).
34. Kaye, A.H., Morstyn, G., Gardner, I. & Pyke, K. Development of a xenograft glioma model in mouse brain. *Cancer Res.* **46,** 1367-1373 (1986).
35. Inazawa, J. et al. Precise localization of the human gene encoding cell adhesion kinase β (CAKβ/PYK2) to chromosome 8 at p21.1 by fluorescence in situ hybridization. *Hum. Genet.* **98,** 508-510 (1996).
36. Klapper, L.N., Kirschbaum, M.H., Sela, M. & Yarden, Y. Biochemical and clinical implications of the ErbB/HER signaling network of growth factor receptors. *Adv Cancer Res.* **77,** 25-79 (2000).
37. Engqvist-Goldstein, Å.E.Y., Kessels, M.M., Chopra, V.S., Hayden, M.R. & Drubin, D.G. An actin-binding protein of the sla/huntington interacting protein 1 family is a novel component of clathrin-coated pits and vesicles. *J. Cell Biol.* **147,** 1503-1518 (1999).
38. Sorkina, T., Bild, A., Tebar, F. & Sorkin, A. Clathrin, adaptors and eps15 in endosomes containing activated epidermal growth factor receptors. *J. Cell. Sci.* **112,** 317-327 (1999).
39. Damer, C.K. & O'Halloran, T.J. Spatially regulated recruitment of clathrin to the plasma membrane during capping and cell translocation. *Mol. Biol. Cell* **11**, 2151-2159 (2000).
40. Gaidarov, I., Santini, F., Warren, R.A. & Keen, J.H. Spatial control of coated-pit dynamics in living cells. *Nature Cell. Biol.* **1,** 1-7 (1999).
41. Ohmori, T., Yatomi, Y., Asazuma, N., Satoh, K. & Ozaki, Y. Involvement of proline-rich tyrosine kinase 2 in platelet activation: tyrosine phosphorylation mostly dependent on αIIbβ3 integrin and protein kinase C, translocation to the cytoskeleton and association with Shc through Grb2. *Biochem. J.* **347,** 561-569 (2000).
42. Sayed, M.R., Sheid, M.P., Stevens, C.M. & Durino, V. Thrombin-stimulated phosphatidylinositol3-kinase activity in platelets is associated with activation of PYK2 tyrosine kinase: Activation of both enzymes is aggregation independent. *J. Cell. Physiol.* **183,** 314-320 (2000).
43. Brunet, A. A. et al. Akt promotes cell survival by phosphorylating and inhibiting a forkhead transcription factor. *Cell* ***96,*** 857-868 (1999).
44. Nguyen, D.H. et al. Myosin light chain kinase functions downstream of Ras/ERK to promote migration of urokinase-type plasminogen activator-stimulated cells in an integrin-selective manner. *J. Cell. Biol.* **146,** 149-164 (1999).
45. Chun-Ming, T. et al. Enhancement of chemosensitivity by Tyrphostin AG825 in high-p185^{neu} expressing non-small cell lung cancer cells. *Cancer Res.* **56,** 1068-1074 (1996).
46. Seedorf, K. et al. Dynamin binds to SH3 domains of phospholipase C gamma and GRB-2. *J. Biol. Chem.* **269,** 16009-16014 (1994).
47. Weber, I. et al. Cytokinesis mediated through the recruitment of cortexillins into the cleavage furrow. *EMBO J.* **18,** 586-594 (1999).
48. Chen, C. & Okayama, H. High efficiency transformation pf mammalian cells by plasmid DNA. *Mol. Cell. Biol.* **7,** 2745-2752 (1987).
49. Alessi, D.R. et al. Assay and expression of mitogen-activated protein kinase, MAP kinase kinase, and Raf. *Methods Enzymol.* **255,** 279-290 (1995).
50. Morgan, S.J., Smith, A.D. & Parker, P. Purification and characterization of bovine brain type I phosphatidylinositol kinase. *Eur J Biochem.* **191**,761-767 (1990).
51. Plowman, G.D. et al. Molecular Cloning and expression of another epidermal growth factor receptor-related gene. *Proc. Natl. Acad. Sci. USA* **87,** 4905-4909 (1990).
52. Baselga, J. Current and planned clinical trials with trastuzumab. *Semin. Oncol.* **27,** 27-32 (2000).
53. Monia, B.P. Antisense approaches for the treatment of cancer. *Cancer Invest.* **18,** 635-650 (2000).

## Claims

1. Use of a HER3 protein as a target for the modulation of the mitogen-activated protein (MAP) kinase pathway.

2. Use of a nucleic acid encoding a HER3 protein or a nucleic acid complementary thereto as a target for the modulation of the MAP kinase pathway.

3. The use of claim 1 or 2 comprising reducing the amount and/or activity of a HER3 protein.

4. The use of claim 3 comprising reducing the expression of HER3.

5. The use of claim 3 comprising the introduction of a HER3 protein inhibitor to a target cell or a target organism.

6. The use of any one of claims 1-5 comprising an inhibition of the phosphorylation of the PYK2 protein.

7. The use of any one of claims 1-6 for the manufacture of an agent for the diagnosis, prevention or treatment of a MAP kinase pathway associated disorder.

8. The use of any one of claims 1-7 for the diagnosis, prevention or treatment of a PYK2 kinase phosphorylation associated disorder.

9. The use of claim 7 or 8 wherein the disorder is a hyperproliferative disease.

10. The use of claim 9 wherein said disorder is selected from inflammatory processes and tumors.

11. The use of claim 10 for the inhibition of tumor invasion particularly in gliomas.

12. Use of a PYK2 protein as a target for the modulation of the MAP kinase pathway.

13. Use of a nucleic acid encoding an PYK2 protein or a nucleic acid complementary thereto as a target for the modulation of the MAP kinase pathway.

14. The use of claim 1 or 2 comprising reducing the amount and/or activity of an PYK2 protein.

15. The use of claim 3 comprising reducing the expression of PYK2.

16. The use of claim 3 comprising the introduction of a PYK2 protein inhibitor to a target cell or a target organism.

17. The use of any one of claims 12-16 comprising an inhibition of the phosphorylation of the PYK2 protein.

18. The use of any one of claims 12-17 for the manufacture of an agent for the diagnosis, prevention or treatment of a MAP kinase pathway associated disorder.

19. The use of any one of claims 12-18 for the diagnosis, prevention or treatment of a PYK2 kinase phosphorylation associated disorder.

20. The use of claim 18 or 19 wherein the disorder is a hyperproliferative disease.

21. The use of claim 20 wherein said disorder is selected from inflammatory processes and tumors.

22. The use of claim 21 for the inhibition of tumor invasion particularly in gliomas.

23. A method of identifying novel modulators of MAP kinase activity comprising screening for substances capable of inhibiting HER3 phosphorylation and/or HER3 kinase activity.

24. A method of identifying novel modulators of MAP kinase activity comprising screening for substances capable of inhibiting PYK2 phosphorylation and/or PYK2 kinase activity.
